Europäisches Patentamt

European Patent Office

Office européen des brevets

(11) Publication number: **0 475 457 A1**

# EUROPEAN PATENT APPLICATION

(21) Application number: **91117364.9**

(22) Date of filing: **25.09.90**

(51) Int. Cl.⁵: **C12M  3/02**, C12N 5/02, //B04B5/04

This application was filed on 11 - 10 - 1991 as a divisional application to the application mentioned under INID code 60.

(30) Priority: **27.09.89 JP 249269/89**
**24.04.90 JP 106444/90**

(43) Date of publication of application:
**18.03.92 Bulletin  92/12**

(60) Publication number of the earlier application in accordance with Art.76 EPC: **0 420 153**

(84) Designated Contracting States:
**CH DE FR GB LI**

(71) Applicant: **TEIJIN LIMITED**
**6-7, Minamihommachi 1-chome Chuo-ku**
**Osaka(JP)**

(72) Inventor: **Tokashiki, Michiyuki**
**1-27-8, Kitanodai**
**Hachioji-shi, Tokyo(JP)**
Inventor: **Hamamoto, Kimihiko**
**Teijin Tama Apartment 142, 3-18-4,**
**Tamadaira**
**Hino-shi, Tokyo(JP)**
Inventor: **Ishimaru, Kenji**
**1-28-4-203, Ozu-cho**
**Iwakuni-shi, Yamaguchi-ken(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) Method of separating animal cells from animal cell-containing suspension using a centrifugal separator, and method of culturing animal cells in suspension.

(57) A method of separating animal cells and an aqueous solution from a living animal cell-containing aqueous suspension, which comprises

(A) feeding a living animal cell-containing aqueous suspension from the feed opening of the liquid fluid in the rotating centrifugal separator, meanwhile withdrawing the aqueous solution separated from the living animal cells via the discharge opening of the liquid fluid, and accumulating the animal cells in the space of the centrifugal separator,

(B) then feeding a liquid carrier immiscible with water, having a higher density than the animal cells and the aqueous solution and not inhibiting the growth of the animal cells from the feed opening of the liquid fluid in the rotating centrifugal separator, withdrawing the living animal cells together with the remaining aqueous solution by pushing them out with the liquid carrier from the discharge opening of the liquid fluid to thereby obtain the living animal cells, and

(C) further feeding a fresh or spent medium solution from the feed opening of the liquid fluid in the rotating centrifugal separator, and withdrawing the liquid carrier from the discharge opening of the liquid fluid to separate the liquid carrier.

EP 0 475 457 A1

DETAILED DESCRIPTION OF THE INVENTION

FIELD OF INDUSTRIAL UTILIZATION

This invention relates to a method of separating animal cells from an animal cell-containing suspension using a centrifugal separator, and a method of culturing animal cells in suspension using centrifugal separator.

PRIOR ART

To prevent the growth of cells from stopping at a relatively low cell density and to culture the cells in large quantities at a high density in suspension, there has been generally proposed a so-called perfusion method comprising culturing cells while supplying a fresh culture fluid to a culture tank and meanwhile discharging the spent culture fluid containing a growth-inhibitory substance out of a culture tank (Annual Reports on Fermentation Processes, vol. 6).

In culturing the animal cells by this method, it is important to efficiently separate the spent culture fluid from living cells in the suspension, discharge the spent culture fluid out of the culture tank and maintain the growth environment for the cells in the culture tank under optimum conditions. Various filters or other systems have been proposed for the separation of living cells and the spent culture fluid. None of them, however, have proved to be entirely satisfactory as an industrial device for culturing cells in large quantities because of blockage of the filters or complexity of the structure of the device.

In Japanese Laid-open Patent Application No. 252558/1988, an apparatus for separating animal cells and a method of culturing cells by a perfusion system suited for culturing cells in large quantities at a high density are proposed by the present inventors. Said proposal comprises a centrifugal separating device and a separating system for continuously or intermittently separating living cells and a spent culture fluid from a suspension culture fluid utilizing a centrifugal force. However, there is a fear of damages to cells owing to a pressure difer ence in the centrifugal separating device occuring when the cells pass through the liquid of a high density used in withdrawing the cells, and this proposal cannot be said to apply to all kinds of cells.

Moreover, since only part of centrifugal separating device can be utilized in the above proposal, the volumetric efficiency is low and the device is therefore not suited to separate large quantities of animal cells within a fixed period of time.

On the other hand, the separation of the animal cells from the animal cell-containing suspension is widely utilized in not only the aforesaid suspension culture but also the separation of cells from bloods of mammals, and is therefore an important technology.

OBJECTS OF THE INVENTION

A first object of this invention is to provide a method of driving and operating a centrifugal separator for separating animal cells from the suspension.

A second object of this invention is to provide an industrially advantageous method of culturing animal cells in suspension using the centrifugal separator.

A third object of this invention is to provide a method of culturing animal cells in suspension at a high density using the centrifugal separator for obtaining useful active proteins produced from the animal cells.

The other objects of this invention will be clarified from the following description.

CONSTRUCTION OF THE INVENTION

According to the study of the present inventors, the objects and advantages of this invention are found to be achieved by a method (I) of separating animal cells and an aqueous solution from a living animal cell-containing aqueous suspension, which comprises

(A) feeding a living animal cell-containing aqueous suspension from the feed opening of the liquid fluid in the rotating centrifugal separator, meanwhile withdrawing the aqueous solution separated from the living animal cells via the discharge opening of the liquid fluid, and accumulating the animal cells in the space of the centrifugal separator,

(B) then feeding a liquid carrier immiscible with water, having a higher density than the animal cells and the aqueous solution and not inhibiting the growth of the animal cells from the feed opening of the liquid fluid in the rotating centrifugal separator, withdrawing the living animal cells together with the remaining aqueous solution by pushing them out with the liquid carrier from the discharge opening of the liquid fluid to thereby obtain the living animal cells, and

(C) further feeding a fresh or spent medium solution from the feed opening of the liquid fluid in the rotating centrifugal separator, and withdrawing the liquid carrier from the discharge opening of the liquid fluid to separate the liquid carrier.

Further, according to the study of the present inventors, the other objects and advantages of this invention are found to be achieved by a method (II) of culturing animals in suspension, which comprises,

(a) culturing living animal cells in suspension in a culture tank,

(b) withdrawing a portion of the living animal cell-containing suspension culture fluid from the culture tank,

(c) feeding the withdrawn suspension culture fluid from the feed opening of the liquid fluid in the rotating centrifugal separator for a certain period of time, meanwhile withdrawing the culture fluid separated from the living cells via the discharge opening of the liquid fluid, and accumulating the animal cells in the space of the centrifugal separator,

(d) then feeding a liquid carrier immiscible with water, having a higher density than the animal cells and the culture fluid and not inhibiting the growth of the animal cells from the feed opening of the liquid fluid in the centrifugal separator, and withdrawing the living animal cells by pushing them out together with the culture fluid from the discharge opening of the liquid fluid,

(e) returning at least a portion of the withdrawn animal cells to the culture tank for step (a), and

(f) further feeding a fresh culture fluid or the culture fluid withdrawn in step (c) from the feed opening of the liquid fluid in the centrifugal separator, and withdrawing the liquid carrier from the discharge opening of the liquid fluid to recover the liquid carrier from the discharge opening of the liquid fluid by separation.

## BRIEF DESCRIPTION OF DRAWINGS

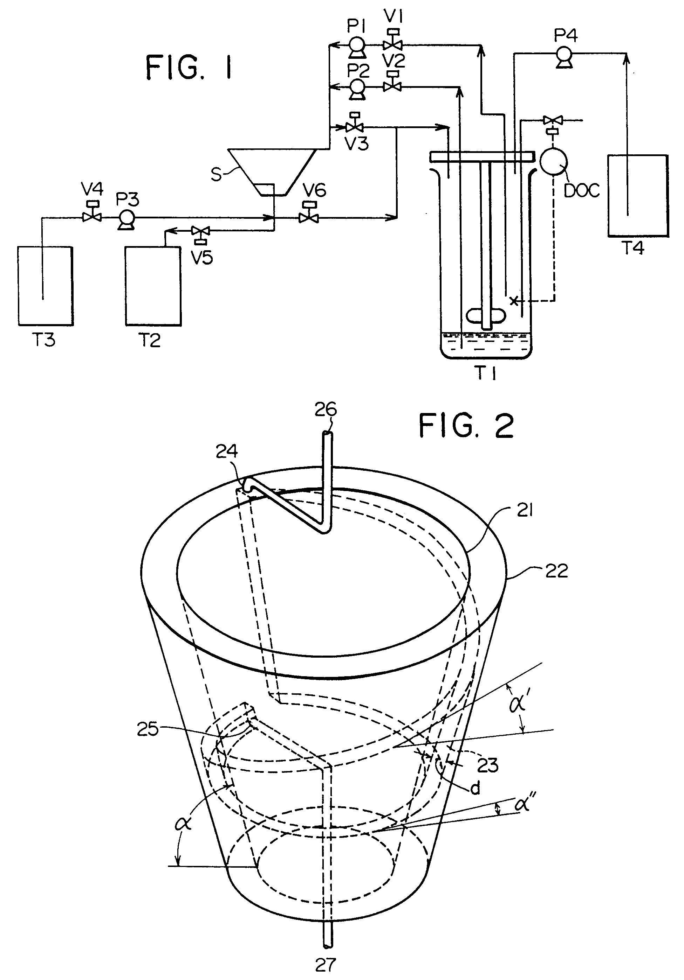

Figure 1 is a rough view of a series of devices suitable for practicing a method of culturing animal cells in suspension in accordance with this invention.

In Figure 2, an inside peripheral wall 21 and an outside peripheral wall 22 form a peripheral slit 23. Shown at 24 is an opening for feeding the suspension culture fluid and also for withdrawing the liquid having a higher density. Shown at 25 is an opening for withdrawing the cell-free mother liquor and the culture fluid containing the separated cells. Nozzles 26 and 27 are also provided for feeding into the rotor and/or withdrawal from the rotor.

The angle $\alpha$ between the slit surface and the centrifugal direction in Figure 2 is preferably 50 to 90°, and the depth $\underline{d}$ of the peripheral slit is preferably 3 to 5 mm. The surface of the inside surface of the peripheral slit is desirably treated so as to render it hydrophobic. This treatment may be suitably carried out by coating it with a fluorine-containing resin such as polytetrafluoroethylene.

The angle $\alpha'$ between the upper side of the peripheral slit 23 and the centrifugal direction in Figure 2 is preferably 5 to 10°, and the angle $\alpha''$ between the lower side of the slit and the centrifugal direction is preferably 0 to 5°.

In the separating method [I] of this invention, the "living animal cell-containing aqueous suspension" being separated can be any solution containing the animal cells in suspension, and its origin is not limited in particular. It may be a suspension withdrawn from a culture tank in suspension culture of animal cells, or body fluids of mammals, especially blood.

The operation of the culturing method [II] in this invention will be explained hereinafter. Said method is not substantially different from the separating method [I]. That is, steps (c), (d) and (f) in the method [II] correspond to steps (A), (B) and (C) in the method [I].

The method [II] of culturing animal cells in accordance with this invention is applicable to the culturing of animal cells in suspension (suspension culture). The suspension culture denotes a method whereby animal cells are cultured while they are suspended in an aqueous medium.

The animal cells to which the culturing method of this invention can be applied are those which can grow or proliferate in suspension. They include not only natural animal cells, but also cells modified artificially or by gene manipulation such as hybridoma cells. Or they may be cells derived from lymphatic cells which produce lymphokine, diploid cells producing biologically active substances such as interferon (IFN), or cells producing various monoclonal antibodies.

The present invention is especially suitable for obtaining monoclonal antibodies at high densities by culturing monoclonal antibody-producing cells.

In step (a) of the method [II] of this invention, the animal cells are cultured in suspension in a culture tank. The culture medium used in the suspension culture is an aqueous medium consisting substantially of water. The aqueous medium contains various additives ordinarily used in the culturing of animal cells, for example inorganic salts, vitamins, coenzymes, glucose, amino acids, antibiotics, and growth promoting factors.

Serum may be added to the culture fluid. But a serum-free culture medium may also be used as the culture fluid. The use of the serum-free culture medium is economically advantageous, and therefore desirable.

The culture tank that can be used in this invention at least comprises an opening for feeding animal cells, an opening for introducing a fresh culture medium, a tube for introduction of air, an agitator, and a conduit for withdrawing the suspension culture fluid. Such a culture tank may be an ordinary culture tank.

In step (b), a portion of the suspension culture fluid containing animal cells is withdrawn from the culture tank, for example through a culture fluid withdrawal conduit. The withdrawal of the suspension culture fluid can be carried out continuously or intermittently. Desirably, a fresh supply of the makeup culture medium is introduced into the culture tank continuously or intermittently in an amount corresponding to the amount of the withdrawn suspension culture fluid containing animal cells. As a result, the concentration of substances which inhibit (adversely affect) the growth of the animal cells and consist mainly of metabolites of the animal cells in the suspension culture fluid within the culture tank can be always maintained at a considerably low level. Thus, the density of the grown animal cells in the suspension culture fluid can be increased.

The amount of useful metabolites produced with the growth of the animal cells can be increased by an amount corresponding to the increased density of the grown animal cells.

By maintaining the concentration of the substances which inhibit the growth of the animal cells at a low level with a sufficient control, the culturing can be continued while maintaining an increased density of the grown animal cells over an extended period of time, and the useful metabolites can be produced in increased amounts.

The above culturing method is characterized by accumulating the living animal cells in the centrifuging space of the centrifugal separator in step (c), and then pushing and withdrawing the accumulated living animal cells together with the mother liquor by the liquid medium immiscible with water, having a higher density than the animal cells and the culture fluid and not hindering the growth of the animal cells in step (d).

In step (c), to accumulate the living animal cells, it is necessary to continuously feed the suspension culture fluid withdrawn in step (b) into the centrifuging space of the centrifugal separator for a certain period of time and to continuously withdraw the mother liquor separated from the living animal cells for a certain period of time. Specifically, since the suspension culture fluid withdrawn in step (b) has a low concentration of the animal cells, it is fed into the centrifuging space until the animal cells are accumulated in a desired amount. The following operating conditions for the centrifugal separator are desirable.

(1) $\theta \leq 300$,

(2) $\bar{Z} \times \theta \leq 3 \times 10^4$,

(3) $Q/S.\bar{Z} \leq 0.3$, and

(4) $5 \leq \bar{Z} \leq 2,000$

wherein

$\theta$ is the average residence time (minutes) of the animal cells in the centrifugal separator,

$\bar{Z}$ is a centrifuging effect,

Q is the amount (ml/min.) of the suspension culture fluid supplied to the centrifugal separator per unit time, and

S is the sedimentation area ($cm^2$) when the centrifugal force is acting.

The average residence time ($\theta$, minutes) of the animal cells in the centrifugal separator should be limited to not more than 300 minutes. If the residence time exceeds 300 minutes, the survival rate of the animal cells becomes markedly low owing, for example, to the deficiency of oxygen. Preferably, the average residence time ($\theta$) is not more than 150 minutes, especially not more than 60 minutes. Most preferable is not more than 30 minutes but not less than 5 minutes.

For example, in the continuous method comprising continuously feeding the suspension culture fluid into the centrifugal separator and continuously withdrawing the separated animal cells, the average residence ime ($\theta$) of the animal cells in the centrifugal separator is obtained as a quotient of the volume (V, $cm^3$) of the space in which the animal cells in the fed suspension culture fluid can exist under centrifugal conditions, divided by the rate ($Q_C$, $cm^3$/min.) of withdrawing components containing the separated animal cells from the centrifugal separator.

$\bar{Z}$ is the centrifugal effect in the centrifugal separator, and is represented by $r\omega^2/g$ in which r is the distance (cm) from the axis of rotation, $\omega$ is the rotating angular speed (radians/sec.), and g is the acceleration of gravity ($cm/sec^2$). The centrifuging effect, as it were, represents the magnitude of e centrifugal force exerted on the animal cells, and is therefore determined by the position (distance r from the axis of rotation) of the culture fluid feed opening for feeding the suspension culture fluid to be fed to the centrifugal separator for separation. $\overline{Z}$ is in the range of 5 to 2,000. If it is lower than 5, the separating operation is difficult to perform. If it exceeds 2,000, the centirifugal force on the animal cells is too high and the cells are undesirably ruptured markedly. Advantageously, the centrifuging effect ($\overline{Z}$) should be maintained in the range of 10 to 1,000, especially preferably 20 to 300.

The $\overline{Z} \times \theta$ should be maintained at not more than $3 \times 10^4$. If the centrifuging operation is carried out while the $\overline{Z} \times \theta$ value is above the above-specified range, the survival rate of the animal cells decreases gradually by the compaction of the cells themselves. Especially preferably, the $\overline{Z} \times \theta$ value is not more than $2 \times 10^4$.

S ($cm^2$) is the sedimentation area ($cm^2$) under the action of a centrifugal force. S ($cm^2$) is defined as an effective area involved in separation at the site (r cm from the axis of rotation) of the opening for feeding the suspension culture fluid into the centrifugal separator for separation. When a phantom circle at the site of the feed opening at a distance of r from the axis of rotation does not cross the sedimentation surface, it is obtained as a value of 2 rh by the site (r) of the feed opening and the height (h) of the liquid surface of the suspension culture fluid at the position of the feed opening. When the phantom circle crosses the sedimentation surface, the effective area decreases to the area of that portion which ranges to the site where the phantom circle crosses the sedimentation surface, and is obtained by multiplying 2 rh by the ratio of the angle to the point of crossing. It should be understood that the height (h) of the liquid surface mentioned above is a vertical distance from the deepest position of the separating tank of the centrifugal separator.

The value S.$\overline{Z}$ obtained by multiplying the effective area S and the centrifuging effect $\overline{Z}$ is a parameter that shows the separating ability of the centrifugal separator. In the method of this invention, a value obtained by dividing the amount Q (ml/min.) of the suspension culture fluid supplied to the centrifugal separator per unit time by this parameter, i.e. Q/S.$\overline{Z}$, should be limited to not more than 0.3, preferably not more than 0.2, especially preferably not more than 0.1.

By ensuring the operating conditions (1) to (4), step (C) of the method of this invention makes it possible to separate living animal cells efficiently at a very high survival rate from the suspension culture fluid.

The living animal cells separated by the centrifugal separator and accumulated in the centrifuging space is then, in step (d) according to the above method of this invention, pushed out from the discharge opening together with the mother liquor by feeding a liquid carrier immiscible with water, having a higher density than the animal cells and the culture fluid and not inhibiting the growth of the animal cells to the centrifugal separator in operation from the feed opening instead of the suspension culture fluid.

Perfluorocarbons, for example, can be suitably used as the liquid carrier. Advantageously, the fluorocarbons are liquid at room temperatures, and commercially available fluorocarbons can be widely utilized. For example, fluorocarbons used as heat media or an electrically insulating material, and various fluorocarbons used as artificial blood may be used. Specific examples include perfluoroalkanes having at least 8 carbon atoms, perfluorocycloalkanes (such as perfluorodecalin, perfluoromethyldecalin, and perfluoroalkylcyclohexane, and perfluoroalkylcyclohexanes having 3 to 5 carbon atoms in the alkyl moiety), perfluoroalkyltetrahydrofurans having 5 to 7 carbon atoms in the alkyl moiety, perfluoroalkyltetrahydropyrans having 4 to 6 carbon atoms in the alkyl moiety, and perfluoroadamantanes (such as perfluoroadamantane, perfluoromethyladamantane, perfluorodimethyladamantane, perfluoromethylethyladamantane and perfluorodiethyladamantane). These perfluorocarbons may contain various groups, for example a tertiary amino group. They may be used either singly or in combination.

The combination of steps (c) and (d) enables the animal cells to be separated while they are alive. Moreover, since the centrifuging space incessantly undergoes washing with the liquid carrier, the centrifuging space can always be maintained in an environment suited for the growth of the living animal cells and for an industrially advantageous continuous operation.

At least a portion of the living animal cells having the ability to proliferate which have been withdrawn in step (d) are then, in step (e) according to the culturing method of this invention, returned to the culture tank for step (a).

Moreover, in this invention, in step (f), the fresh culture fluid or the culture fluid withdrawn in step (c) is fed from the feed opening of the liquid fluid of the rotating centrifugal separator, and the liquid carrier is withdrawn from the discharge opening of the liquid fluid to recover it from the centrifugal separator by separation.

The culturing method of this invention is an industrially profitable method that exhibits the aforesaid advantages by repeating steps (b) to (f).

A series of suitable devices for practicing the culturing method of this invention will be explained

EP 0 475 457 A1

hereinafter by referring to Figure 1.

Figure 1 is an outline view of an apparaatus suitable for practicing the culturing method of this invention.

In Figure 1, an animal cell-containing suspension culture fluid withdrawn from a culture tank T1 by means of a pump P1 is continuously fed in a centrifugal separator S from a feed opening provided at a site under a higher centrifugal force. By the centrifugal force, the animal cells are separated from the suspension fluid, and the mother liquor separated from the animal cells is withdrawn continuously and aseptically from a discharge opening provided at a site under a lower centrifugal force. Thereafter, from the bottom portion of the culture tank T1, a liquid carrier which has no affinity for either the cells or the culture fluid and has a higher density than these is withdrawn by a pump P2, and fed in a fixed amount from the feed opening provided at that site of the centrifugal separator S which is under a higher centrifugal force. As a result, the culture fluid containing the separated animal cells is aseptically withdrawn from the discharge opening of the centrifugal separator which is at a site under a lower centrifugal force, and returned to the culture tank T1. Thereafter, a cell-free medium is withdrawn from the tank T3 via the pump P3, and fed from the discharge opening of the centrifugal separator S, whereby the liquid carrier having a higher density in the centrifugal separator is again returned to the culture tank T1 and the inside of the rotor is replaced by the culture medium. A tank T4 contains a cell-free fresh culture medium which is continuously or intermittently fed into the tank T1 by means of a pump P4 so that the liquid level in the culture tank T1 becomes constant.

By performing the above operation continuously or intermittently without stopping the centrifugal separator, the animal cells can efficiently be separated at a very high survival rate. Needless to say, valves V1 to V6 require an opening-closing operation suitable for the above operation.

As is apparent from the foregoing explanation, the culturing method of this invention is said to be a method which employs the centrifugal separator and uses the liquid carrier immiscible with water, having a higher density than the animal cells and the aqueous solution and not inhibiting the growth of the animal cells, to thereby exhibit the functions thereof most effectively.

That is, in the culturing method of this invention shown in Figure 1, the liquid carrier in the bottom portion of the culture tank is introduced into the centrifugal separator S via the pump P2, the living animal cells accumulated in the centrifugal separator S are thereby pushed out and returned to the culture tank together with the cells. By performing the operation while driving the centrifugal separator, the liquid carrier stays in the centrifugal separator, and the cells which have so far resided therein are spontaneously discharged owing to difference in specific gravity. On this occasion, part or the whole of the liquid carrier is sometimes introduced into the culture tank T1, and therefore the liquid carrier phase is formed at the bottom portion of the culture tank. It will be seen that the above recycling use of the liquid carrier phase formed in the bottom portion of the culture tank is quite advantageous to industrial-scale culturing.

Since the cells do not pass through the liquid carrier having a higher specific gravity by this operation, the exertion of the unnecessary pressure on the cells is avoidable, and damage to the cells is preventable.

According to this invention, the time during which the animal cells pass through the liquid carrier can be reduced to zero or to a very short one. The damage to the animal cells is little if the pressure substantially exerted on the animal cells is 0.2 kgf/cm$^2$ or less. The animal cells are therefore allowed to pass through a liquid carrier having a pressure within this pressure range.

Moreover, by operating this apparatus as described above, the concentration of substances which inhibit the growth of the animal cells accumulated successively within the culture tank as the culturing proceeds can be maintained at low levels.

The following Examples illustrate this invention more specifically.

EXAMPLE

(1) Culture device

A culture system having a cell separating unit of the type shown in Figure 1 was used. The culture tank T1 was a stirred culture tank of glass having a total capacity of 15 liters and designed to receive 10 liters as a net volume of the culture medium.

Into the bottom portion of the culture tank, 900 ml of a perfluorocarbon (FLUORINERT® FC-40®, a product of 3M, U. S. A.) was put.

The centrifugal separator was one equipped with a rotor having a sedimentation area S of 130 cm$^2$, an effective volume of 75 ml and a rotation radius of 10 cm, as shown in Figure 2. Pumps P1, P2 and P3 were peristaltic pumps.

(2) Culture medium

A 2:1:1 mixture of RPMI 1640 medium, HAM 12 medium and Dulbecco' modified Eagle medium (to be referred to as RDF) was used as a base medium.

A medium obtained by adding 9 micrograms/ml of insulin, 10 micrograms/ml of transferrin, 10 micrograms/ml of ethanolamine and $2 \times 10^{-6}$ mole/liter of selenous acid to a base medium was used.

(3) Method and results of culturing

The culture system was sterilized in advance by autoclaving. Then, 10 liters of the culture medium sterilized by filtration was fed into the culture tank, and mouse-human hybridoma X87 cells obtained by fusing mouse myeloma P3Ul cells and human B cells were seeded so that the cell density became 6.2 x $10^5$ cells/ml. These hybridoma cells produce IgG. Oxygen gas containing 5 % of carbon dioxide was introduced into the culture tank through a blow nozzle while the concentration of dissolved oxygen in the culture medium was automatically controlled to 3 ppm. The culture fluid in the culture tank was maintained at 37°C. A marine-type stirring vane was attached to the cultivation tank, and operated at a stirring speed of 30 rpm.

For a day after the seeding, the culturing was carried out batchwise. As shown in Table 1, the cell density reached 9.8 x $10^5$ cells/ml on the second day after the start of culturing (after the lapse of 24 hours). This density was determined to be the highest in batchwise culture, and perfusion culture using the centrifugal separator was started. Specifically, the centrifugal separator charged with the filtration-sterilized culture fluid was driven, and the rotation speed of the centrifugal separator was adjusted so that the centrifuging effect became 80G.

Then, 500 ml of the culture fluid was sent to the centrifugal separator over 10 minutes at a rate of 50 ml/min. The mother liquor separated from the cells by the centrifugal separator flowed into the reservoir tank T2 for the spent culture medium and was stored. The density of the cell therein was 1.0 x $10^4$ cells/ml.

After the foregoing operation was terminated, pump P-2 was driven to send 120 ml of the perfluorocarbon at the bottom of the culture tank T1 at a rate of 30 ml/min. By this operation, all the cells residing in the centrifugal rotor were discharged from the centrifugal separator together with the perfluorocarbon, and returned to the culture tank T1. At this time, the pressure exerted on the cells within the centrifugal rotor was 0.05 kgf/cm$^2$ at the highest.

Thereafter, pumps P1 and P2 were stopped, and pump P3 was driven to send the fresh medium in T3 to the centrifugal separator S and return the perfluorocarbon in the centrifugal separator to the culture tank T1.

The culturing was continued by automatically performing this operation once every period of time shown in Table 1. A fresh supply of the culture medium was fed continuously into the culture tank so that the liquid level in the culture tank became constant on an average.

The experimental results are shown in Table 16 together with some of the experimental conditions.

Table 1

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $6.2 \times 10^5$ | 0 |
| 1 | 1.2 | 1.0 | $9.8 \times 10^5$ | 11 |
| 2 | 1.2 | 1.0 | $1.6 \times 10^6$ | 8 |
| 3 | 1.2 | 1.0 | $2.3 \times 10^6$ | 8 |
| 4 | 1.2 | 1.0 | $3.4 \times 10^6$ | 12 |
| 5 | 0.6 | 2.0 | $4.8 \times 10^6$ | 10 |
| 6 | 0.6 | 2.0 | $6.8 \times 10^6$ | 14 |
| 7 | 0.6 | 2.0 | $8.9 \times 10^6$ | 22 |
| 8 | 0.6 | 2.0 | $9.8 \times 10^6$ | 29 |
| 9 | 0.6 | 2.0 | $1.1 \times 10^7$ | 38 |
| 10 | 0.6 | 2.0 | $1.0 \times 10^7$ | 45 |
| 11 | 0.6 | 2.0 | $1.2 \times 10^7$ | 48 |
| 12 | 0.6 | 2.0 | $1.1 \times 10^7$ | 54 |
| 13 | 0.6 | 2.0 | $1.2 \times 10^7$ | 53 |
| 14 | 0.6 | 2.0 | $1.2 \times 10^7$ | 56 |

**Claims**

1. A method of separating animal cells and an aqueous solution from a living animal cell-containing aqueous suspension, which comprises

   (A) feeding a living animal cell-containing aqueous suspension from the feed opening of the liquid fluid in the rotating centrifugal separator, meanwhile withdrawing the aqueous solution separated from the living animal cells via the discharge opening of the liquid fluid, and accumulating the animal cells in the space of the centrifugal separator,

   (B) then feeding a liquid carrier immiscible with water, having a higher density than the animal cells and the aqueous solution and not inhibiting the growth of the animal cells from the feed opening of the liquid fluid in the rotating centrifugal separator, withdrawing the living animal cells together with the remaining aqueous solution by pushing them out with the liquid carrier from the discharge opening of the liquid fluid to thereby obtain the living animal cells, and

   (C) further feeding a fresh or spent medium solution from the feed opening of the liquid fluid in the rotating centrifugal separator, and withdrawing the liquid carrier from the discharge opening of the liquid fluid to separate the liquid carrier.

2. The method of claim 1 wherein the living animal cell-containing aqueous suspension is an artificially cultured suspension.

3. The method of claim 1 wherein the living animal cell-containing aqueous suspension is a blood from a mammal.

4. The method of any one of claims 1 to 3 wherein the liquid carrier is a fluorocarbon.

5. The method of any one of claims 1 to 4 wherein the centrifugal separator is operated under the following conditions:

   (1) $\theta \leq 300$,
   (2) $\overline{Z} \times \theta \leq 3 \times 10^4$,
   (3) $Q/S.\overline{Z} \leq 0.3$, and
   (4) $5 \leq \overline{Z} \leq 2000$

   wherein

   $\theta$ is an average residence time (minutes) of the animal cells in the centrifugal separator,

   $\overline{Z}$ is a centrifuging effect,

   $Q$ is the amount (ml/min.) of the suspension culture fluid supplied to the centrifugal separator per unit time, and

   $S$ is the sedimentation area ($cm^2$) when the centrifugal force is acting.

6. A method of culturing animal cells in suspension, which comprises,

   (a) culturing living animal cells in suspension in a culture tank,

   (b) withdrawing a portion of the living animal cell-containing suspension culture fluid from the culture tank,

   (c) feeding the withdrawn suspension culture fluid from the feed opening of the liquid fluid in the rotating centrifugal separator for a certain period of time, meanwhile withdrawing the culture fluid separated from the living cells via the discharge opening of the liquid fluid, and accumulating the animal cells in the space of the centrifugal separator,

   (d) then feeding a liquid carrier immiscible with water, having a higher density than the animal cells and the culture fluid and not inhibiting the growth of the animal cells from the feed opening of the liquid fluid in the centrifugal separator, and withdrawing the living animal cells by pushing them out together with the culture fluid from the discharge opening of the liquid fluid,

   (e) returning at least a portion of the withdrawn animal cells to the culture tank for step (a), and

   (f) further feeding a fresh culture fluid or the culture fluid withdrawn in step (c) from the feed opening of the liquid fluid in the centrifugal separator, and withdrawing the liquid carrier from the discharge opening of the liquid fluid to recover the liquid carrier from the discharge opening of the liquid fluid by separation.

9

**7.** The method of claim 6 wherein steps (b) to (f) are repeated.

**8.** The method of claim 6 or 7 wherein the animal cells are hybridoma cells.

**9.** The method of any one of claims 6 to 8 wherein the liquid carrier is a fluorocarbon.

**10.** The method of any one of claims 6 to 9 wherein the centrifugal separator is operated under the following conditions:

(1) $\theta \leq 300$,

(2) $\overline{Z} \times \theta \leq 3 \times 10^4$,

(3) $Q/S.\overline{Z} \leq 0.3$, and

(4) $5 \leq \overline{Z} \leq 2000$

wherein

$\theta$ is an average residence time (minutes) of the animal cells in the centrifugal separator,

$\overline{Z}$ is a centrifuging effect,

Q is the amount (ml/min.) of the suspension culture fluid supplied to the centrifugal separator per unit time, and

S is the sedimentation area ($cm^2$) when the centrifugal force is acting.

FIG. 1

FIG. 2

11

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN<br>vol. 13, no. 42 (C-564)(3390) 30 January 1989<br>& JP-A-63 240 783 ( TEIJIN LTD ) 6 October 1988<br>* abstract * | 1-10 | C12M3/02<br>C12N5/02<br>//B04B5/04 |
| X | EP-A-0 229 289 (TEIJIN LTD)<br>* page 8, line 5 - line 52; claims * | 1-10 | |

TECHNICAL FIELDS
SEARCHED (Int. Cl.5)

C12M
B04B

The present search report has been drawn up for all claims

| Place of search | Date of completion of the search | Examiner |
|---|---|---|
| THE HAGUE | 18 NOVEMBER 1991 | RYCKEBOSCH A.O. |

EPO FORM 1503 03.82 (P0401)